(19) Europäisches Patentamt
European Patent Office
Office européen des brevets

(11) EP 1 738 744 B1

# (12) EUROPÄISCHE PATENTSCHRIFT

(45) Veröffentlichungstag und Bekanntmachung des Hinweises auf die Patenterteilung:
**03.11.2010 Patentblatt 2010/44**

(21) Anmeldenummer: **06116031.3**

(22) Anmeldetag: **26.06.2006**

(51) Int Cl.:
***A61K 8/49*** (2006.01) ***A61Q 17/02*** (2006.01)
***A01N 47/16*** (2006.01) ***A01N 25/02*** (2006.01)
***A01N 25/34*** (2006.01)

(54) **Hautfreundliches Insektenabwehrmittel**

Skin-friendly insect repellent

Produit insectifuge bien toléré par la peau

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **27.06.2005 DE 102005030016**

(43) Veröffentlichungstag der Anmeldung:
**03.01.2007 Patentblatt 2007/01**

(73) Patentinhaber: **Beiersdorf Aktiengesellschaft**
**20253 Hamburg (DE)**

(72) Erfinder:
- **Schulz, Jens**
  **22869 Schenefeld (DE)**
- **Von der Fecht, Stephanie**
  **22869 Schenefeld (DE)**
- **Nielsen, Jens**
  **25448 Henstedt-Ulzburg (DE)**
- **Kröpke, Rainer**
  **22869 Schenefeld (DE)**

(56) Entgegenhaltungen:
**WO-A-02/43490** **WO-A-02/43656**
**WO-A-03/020232** **DE-A1- 10 349 666**

Bemerkungen:
Die Akte enthält technische Angaben, die nach dem Eingang der Anmeldung eingereicht wurden und die nicht in dieser Patentschrift enthalten sind.

EP 1 738 744 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft eine kosmetische Zubereitung enthaltend einen oder mehrere Repellent-Wirkstoffe mit einem Dampfdruck von mindestens 3,2 $10^{-4}$ hPa bei 20 °C und eine oder mehrere Substanzen mit einem log P-Wert von -2,5 bis +2,5.

Insektenabwehrmittel (Insektenvertreibungsmittel, Insektenschutzmittel, Repellentien, Repellents) sind Präparate, die zur Abwehr und/oder Vertreibung von Insekten, aber auch Zecken und Milben äußerlich angewendet werden und verhindern sollen, dass diese auf der Haut aktiv werden. Insektenabwehrmittel sollen die Haut vor Belästigung durch Blut saugende oder beißende Insekten und andere Parasiten und/oder Lästlinge schützen, indem sie diese abwehren, bevor sie sich auf der Haut niederlassen. Die Mittel wirken dementsprechend nicht als Kontaktgifte, sondern nur als Abwehrmittel, da sie die Tiere nicht töten, sondern lediglich vertreiben.

Demgemäß sind im Sinne der vorliegenden Erfindung unter dem Begriff "Insektenschutzmittel" nicht nur solche Präparate zu verstehen, die gegen Insekten (insbesondere Mücken) wirksam sind. Vielmehr gilt das nachstehend Gesagte selbstverständlich auch für solche Präparate, die andere Blut saugende oder beißende Parasiten und/oder Lästlinge (z.B. Spinnen, Flöhe, Milben) abwehren oder vertreiben, auch wenn dies im Einzelfall nicht erwähnt sein mag.

Schon seit Urzeiten werden die Menschen von stechenden oder beißenden Insekten oder anderen Parasiten geplagt. Dementsprechend alt ist das Bedürfnis der Menschheit nach Insektenabwehrmitteln. Eine schon seit der Frühgeschichte bekannte Methode, lästigen oder schädlichen Insekten ihren Aufenthalt in der Nähe des Menschen unattraktiv oder unangenehm zu machen, ist das Anzünden von Feuern mit aromatisch oder streng riechenden Kräutern oder Hölzern und starker Rauchentwicklung. Auch die Behandlung der Haut mit stark riechenden Substanzen zur Abwehr von Insekten ist bereits seit der Antike bekannt. Um die letzte Jahrhundertwende war eine Reihe natürlicher etherischer Öle als Insektenabwehrmittel im Gebrauch, so beispielsweise Anisöl, Bergamottöl, Birkenholzteer, Campher, Citronellöl, Eucalyptusöl, Geraniumöl, Kiefernöle, Kokosnußöl, Lavendelöl, Muskatnußöl, Nelkenöl, Orangenblütenöl, Pfefferminzöl, Poleiöle (Pennyroyalöl), Pyrethrum, Thymianöl und Zimtöl.

Wegen ihrer trotz intensiven Geruchs unzureichenden Wirksamkeit und ihrer zum Teil mangelnden Verträglichkeit in höheren Konzentrationen wurden diese Stoffe in heutigen Insektenabwehrmitteln weitgehend durch besser wirksame synthetische Substanzen verdrängt. Es handelt sich dabei überwiegend um hoch siedende Flüssigkeiten oder niedrig schmelzende bzw. sublimierende kristalline Stoffe, die bei Raumtemperatur langsam verdampfen. Die meisten Repellent-Wirkstoffe gehören den Stoffklassen der Amide, Alkohole, Ester und Ether an.

Ein moderner Repellent-Wirkstoff ist beispielsweise der 1-Piperidincarboxylsäure 2-(2-hydroxyethyl)-1-methylpropylester (INN: Icaridin, CAS-Nummer: 119515-38-7, Elincs-Nummer: 423-210-8), der die folgende Struktur

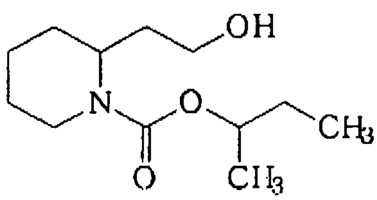

und einen bei 20 °C einen Dampfdruck von 3,4 $10^{-4}$ hPa aufweist. 1-Piperidincarboxylsäure 2-(2-hydroxyethyl)-1-methylpropylester gilt als besonders wirksamer Repelient-Wirkstoff. Insektenabwehrmittel des Standes der Technik, die mit 1-Piperidincarboxylsäure 2-(2-hydroxyethyl)-1-methylpropylester formuliert werden, weisen jedoch eine Reihe von Nachteilen auf. Insbesondere bei Zubereitungen, die gegen Zecken oder die aggressive Anopheles-Mücke wirken sollen, muss der Wirkstoff in hohen Konzentrationen (bis zu 20 Gewichts-% der Zubereitung) eingesetzt werden, um bei der Anwendung eine begrenzte Zeit ausreichend wirksam zu sein. Die Zubereitungen des Standes der Technik haben bei derartig hohen Einsatzkonzentrationen den Nachteil, insbesondere bei Menschen mit empfindlicher oder zu Allergien neigender Haut nicht besonders verträglich zu sein und in Einzelfällen zu Hautreizungen zu führen.

Es war daher die Aufgabe der vorliegenden Erfindung, kosmetische Zubereitungen mit einem Gehalt an 1-Piperidincarboxylsäure 2-(2-hydroxyethyl)-1-methylpropylester zu entwickeln, die besonders hautfreundlich und hautverträglich sind, und ein reduziertes Hautreizungspotential aufweisen.

Überraschend gelöst wird die Aufgabe durch ein Packmittel aus Polyethylen hoher Dichte (HDPE), enthaltend eine kosmetische Zubereitung enthaltend

a) 1-Piperidincarboxylsäure 2-(2-hydroxyethyl)-1-methylpropylester (INN: Icaridin) und

b) eine oder mehrere Substanzen mit einem log P-Wert von -2,5 bis +2,5, gewählt aus der Gruppe mehrwertige Alkohole, lineare Alkohole, verzweigte Alkohole, C5-C10 Alkylalkohole, Polyglycerine.

Zwar kennt der Stand der Technik die DE 103 49 666 A1, WO 03/020232, WO 02/43490. WO 02/43656. doch konnten diese Schriften nicht den Weg zur vorliegende Erfindung weisen.

Ob eine Substanz einen log P-Wert von -2,5 bis +2,5 aufweist, lässt sich erfindungsgemäß mit der folgenden Messmethode bestimmen:

Der Verteilungskoeffzient Log P ist der Quotient der Konzentrationen einer Substanz in der Wasserphase bzw. der Octanolphase nach Einstellung des Verteilungsgleichgewichtes. Die analytische Bestimmung der Konzentrationen wird meist UV-spektroskopisch durchgeführt.

Der Octanol//Wasser-Verteilungskoeffizient (P) einer Substanz "A" ist definiert als (Gleichung 1):

$$P = \frac{\text{Konzentration—Lipidphase}}{\text{Konzentration – Wasserphase}} \Rightarrow \log P = \log \frac{[\text{Octanol}]}{[\text{Wasser}]} \qquad \text{(Gleichung 1)}$$

Log P > 0 => Substanz löst sich besser in Octanol

Log P <0 => Substanz löst sich besser in Wasser

Es können auch direkt die Extinktionen der UV-spektroskopischen Konzentrationsbestimmung in die Gleichung eingesetzt werden. Bei einem Volumenverhältnis Octanol/Wasser 1/1 gilt (Gleichung 2):

$$\text{Log } P = \log \frac{E_0 - E_1}{E_1} \qquad \text{(Gleichung 2)}$$

$E_0$ = Extinktion Maßlösung: $E_1$ = Extinktion der Probe in Wasser ( Maßlösung in Wasser)

Versuchsbeschreibung:

Geräte: Maßkolben für Standardlösung (20-100ml), DC-Kammer (klein), DC-Platten-Alugram, 5 Schütteltrichter, Klemme, Stativring, Zentrifuge, Zentrifugengläser, UV-Spectrometer, Quarzküvetten (1 cm).

Chemikalien: Octanol, Wasser dest., Substanz

Die erhaltene Probe wird mittels Dünnschichtchromatographie auf ihre Reinheit untersucht, da schon geringe Verunreinigungen eine starke Auswirkung auf die Extinktion und damit auf den mit Hilfe der Extinktion ermittelten Log P-Wert haben.

Es wird danach eine Standardldsung (in der Regel $10^{-4}$ M) in dem jeweiligen Puffer hergestellt (Maßkolben), die im Ausschüttelexperiment als auch als Standard für die UV-Messung benutzt wird

Es wird ein UV-Spektrum der Standardlösung aufgenommen (200nm - 300nm Scanmodus).

Die Standardlösung sollte bei der Messung im Maximum eine Extinktion zwischen 0,25 und 1,0 aufweisen.

Ist dies nicht der Fall, ist entsprechend zu verdünnen.

15 ml der Maßlösung werden mit 15 ml des entsprechenden, fehlenden Lösungsmittels in einen Schütteltrichter gefüllt (Volumenverhältnis organische Phase / wässrige Phase, 1/1) und 15 - 20 min. gleichmäßig geschüttelt. Zur Komplettierung der Phasentrennung wird 20 min bei 2500-3000 rpm zentrifugiert.

Alternativ kann das Schütteln der Probe in 2ml-Eppendorfcaps erfolgen. Hierzu werden 1 ml der Maßlösung und 1ml des fehlenden Lösungsmittels 15 - 20 min. in einem "2ml-Eppi' in einem speziellen Schüttler durchmischt, vervollständigt wird die Trennung der beiden Phasen wiederum durch Zentrifugation.

Ein dritte Möglichkeit - und auch die Methode der Wahl - bietet eine spezielle Schüttelapparatur. Hier können mit je 2 (3) ml Octanol und Wasser (bzw. Puffer) befüllte verschließbare Zentrifugenröhrchen eingespannt und für mindestens 20 min. geschüttelt werden. Danach schließt sich wieder der oben beschriebene Zentrifugationschritt an.

Während der Zentrifugation wird die Maßlösung noch einmal gegen das entsprechende Lösungsmittel UV-spektroskopisch vermessen ( Wert = $E_0$ ). Nach Beendigung der Zentrifugation werden gleiche Mengen (Volumen) aus der Wasser bzw. Octanolphase entnommen und gegen das entsprechende Lösemittel UV-spektroskopisch vermessen.

Die erhaltenen Extinktionen können direkt zur Bestimmung des Verteilungskoeffizienten benutzt werden ( s. Gleichung (2)).

Darüber hinaus kann der log P-Wert nach der Methode von *"Hansch und Leo"*, die 1979 veröffentlicht worden ist, berechnet werden.

Die Hansch und Leo Methode beinhaltet hergeleitete Fragmentkonstanten f (Atome oder Atomgruppen) und Strukturfaktoren F (berücksichtigen die räumliche Anordnung des Moleküls Die Berechnung der Verteilungskonstante log Kow verläuft nach Gleichung:

$$\log K = \Sigma F + \Sigma f$$

Die f- und F-Werte wurden hergeleitet aus Hunderten von bekannten Verbindungen. Bei den Fragmentwerten f muss unterschieden werden, ob sie an aliphatische oder aromatische C Atome gebunden sind. Zusätzlich müssen noch weitere 14 Strukturparameter, die Auskunft über die molekulare Beweglichkeit geben, in Betracht gezogen werden.

Literatur

- Seydel, Schaper, Chemische Struktur und biologische Aktivität von Wirkstoffen, 1979, Verlag: Chemie Weinheim
- Strategy of Drug Design: A guide to biological Activity, Purcell, Bass, Clayton, 1973, J. Wiley and Sons
- Rekker, Mannhold, Calculation of Drug Lipophilicity, 1992, Verlag Chemie Weinheim
- Kubiny, H.; QSAR: Hansch Analyses and Related Approaches, Vol 1, Kap. 4.5, 5. 77 ff Brehm, O., Ohlmeyer, I. and Fels, G.; Automatisierte pKa- und logP-Bestimmung. GIT Labor-Fachzeitschrift, 1997, 41, 368-374.


Es ist vorteilhaft im Sinne der vorliegenden Erfindung, wenn die erfindungsgemäße Zuberitung dadurch gekennzeichnet ist, dass die kosmetische Zubereitung Repellent-Wirkstoffe mit einem Dampfdruck von mindestens die kosmetische Zubereitung Repellent-Wirkstoffe mit einem Dampfdruck von mindestens 3.2 $10^{-4}$ hPa bei 20 °C in einer Gesamtkonzentration von 1 bis 40 Gewichts-% und bevorzugt in einer Gesamtkonzentration von 10 bis 20 Gewichts-%, jeweils bezogen auf das Gesamtgewicht der Zubereitung, enthält.

Des weiteren ist es vorteilhaft im Sinne der vorliegenden Erfindung, wenn die erfindungsgemäße Zubereitung dadurch gekennzeichnet ist, dass die kosmetische Zubereitung Substanzen mit einem log P-Wert von -2,5 bis +2,5 in einer Gesamtkonzentration von 0,1 bis 20 Gewichts-% und bevorzugt in einer Gesamtkonzentration von 1 bis 10 Gewichts-%, jeweils bezogen auf das Gesamtgewicht der Zubereitung, enthält.

Vorteilhafte Ausführungsformen der vorliegenden Erfindung sind dadurch gekennzeichnet, dass die erfindungsgemäße Zubereitung, dadurch gekennzeichnet ist, dass das Gewichtsverhältnis der Gesamtmenge an Repellent-Wirkstoffen mit einem Dampfdruck von mindestens $3{,}2 \cdot 10^{-4}$ hPa bei 20 °C zur Gesamtmenge an Substanzen mit einem log P-Wert von -2,5 bis +2,5 von 20 : 1 bis 1: 1 und bevorzugt von 10 : 1 bis 2: 1 beträgt.

Es ist erfindungsgemäß, wenn als Repellent-Vfirkston mit einem Dampfdruck von mindestens 3,2 $10^{-4}$ hPa bei 20 °C 1-Piperidincarboxylsäure 2-(2-hydroxyethyl)-1-methylpropylester (INN: Icaridin) eingesetzt wird.

Es ist erfindungsgemäß, wenn als Substanzen mit einem log P-Wert von -2,5 bis +2,5 eine oder mehrere Verbindungen gewählt aus der Gruppe mehrwertige Alkohole, lineare Alkohole, verzweigte Alkohole, C5-C10 Alkylalkohole, Polyglycerine, eingesetzt werden.

Es ist erfindungsgemäß bevorzugt, wenn als Substanzen mit einem log P-Wert von -2,5 bis +2,5 eine oder mehrere Verbindungen gewählt aus der Gruppe C5-C10 Alkylalkohole und verzweigten Alkohole, eingesetzt werden.

Erfindungsgemäß besonders bevorzugt ist der Einsatz von C5-C10 Alkylalkoholen als Substanz mit einem log P-Wert von -2,5 bis +2.5.

Die erfindungsgemäße kosmetische Zubereitung sind erfindungsgemäß vorteilhaft dadurch gekennzeichnet, das die Zubereitung in Form einer wässrigen oder wässrig-alkoholischen Lösung, einer Emulsion (W/O, OIW, W/S, S/W oder multiple Emulsion), einer Dispersion, einer Pickering-Emulsion vorliegt. Dabei ist es

erfindungsgemäß bevorzugt, wenn die kosmetische Zubereitung in Form einer Emulsion vorliegt.

Die Zubereitung kann erfindungsgemäß vorteilhaft in Form einer dünnflüssigen, sprühfähigen wässrigen oder wässrig-alkoholischen Lösung, in Form eines Gels, als Salbe, Creme oder Lotion (ggf. sprühbar) vorliegen.

Die Zubereitung kann erfindungsgemäß vorteilhaft auch als Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Eine eventuell vorhandene Ölphase kann alle herkömmlichen Bestandteile von in der Kosmetik eingesetzten Öl-, Fett- und Wachskomponenten enthalten.

Als Emulgatoren können alle aus der Kosmetik bekannten Emulgatoren und Emulgatorsysteme eingesetzt werden.

Neben den erfindungsgemäßen Repellent-Wirkstoffen kann die erfindungsgemäße Zubereitung weitere kosmetische Wirk- und Pflegestoffe enthalten, beispielsweise die nach der Kosmetikverordnung zugelassenen UV-Lichtschutzfilter, und Konservierungsmittel bzw. Konservierungshelfer. Derartige Wirkstoffe können erfindungsgemäß vorteilhaft in Konzentrationen von 0,01 bis 30 Gew-%, bezogen auf das Gesamtgewicht der Zubereitung in dieser enthalten sein.

Als weitere Pflegestoffe können insbesondere Niacinamid, Panthenol, Aloe vera, Hammamelis-Extrakt, Polidocanol, Vitamin E, Vitamin E-Derivate, Vitamin A, Vitamin A-Derivate, Vitamin C, Vitamin C-Derivate, Coenzym Q10, Kreatin, Taurin, Alphaglycosylrutin und/oder in Konzentrationen von 0,1 bis 30 Gewichts-%, bezogen auf das Gesamtgewicht der Zubereitung eingesetzt werden.

Erfindungsgemäß besonders bevorzugt enthält die erfindungsgemäße Zubereitung als weitere Bestandteile Alpha-Hydroxysäuren und/oder deren Salze. Dabei werden erfindungsgemäß bevorzugt Milchsäure/Lactate oder Zitronensäure/Citrate in einer Konzentration von 0,01 bis 5 Gewichts-%, bezogen auf das Gesamtgewicht der Zubereitung eingesetzt.

Es ist erfindungsgemäß vorteilhaft, wenn die erfindungsgemäße kosmetische Zubereitung frei ist von Parabenen.

Das erfindungsgemäße Packmittel liegt erfindungsgemäß vorteilhaft in Form einer Tube oder einer Kunststoff-Flasche vor. Dabei sind Kunststoff-Flaschen erfindungsgemäß bevorzugt. Eine erfindungsgemäß besonders bevorzugte Ausführungsform stellen Kunststoff-Flaschen mit aufgesetztem Pumpspender dar, mit welchem die erfindungsgemäße Zubereitung aus dem Packmittel gefördert und ggf. versprüht werden kann.

Erfindungsgemäß ist der Einsatz von "high density polyethylene" d.h. Polyethylen hoher Dichte (HDPE nach DIN 7728, Tl. 1, 01/1988).

Erfindungsgemäß ist insbesondere die Verwendung der erfindungsgemäßen Zubereitung zur Prophylaxe von Mückenstichen und Zeckenbissen.

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

**W/O-Emulsionen**

| | 1 | 2 | 3 | 4 | 5 |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 7,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |

(fortgesetzt)

| | 1 | 2 | 3 | 4 | 5 |
|---|---|---|---|---|---|
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Methylpropandiol | 0,5 | 1,0 | --- | 0,25 | 2,5 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,04 | 0,15 | 0,05 | 0,03 | 0,4 |
| Benzylalkohol | 0,3 | 0,4 | 0,25 | 0,15 | --- |
| Pentylene Glycol | --- | --- | 1,5 | --- | --- |
| Icaridin | 5 | 7,5 | 10 | 12,5 | 30 |
| Wasser | ad 100 | ad 100 | ad 100 | ad 100 | ad 100 |

**W/O-Emulsionen**

| | 6 | 7 | 8 | 9 | 10 |
|---|---|---|---|---|---|
| PEG-30 Dipolyhydroxystearat | --- | 0,5 | 0,25 | --- | 8,0 |
| Lanolin Alcohol | 1,0 | 1,5 | 1,75 | 3,0 | -- |
| Paraffinöl | 12,5 | 10,0 | 8,0 | 5,0 | 12,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Natriumdehydracet | --- | --- | 0,05 | --- | --- |

(fortgesetzt)

| | 6 | 7 | 8 | 9 | 10 |
|---|---|---|---|---|---|
| Kaliumsorbat | 0,3 | 0,4 | 0,25 | 0,15 | --- |
| Polyurethan | 1,5 | --- | 1,0 | 2,5 | 10 |
| Icaridin | 7,5 | 12,5 | 17,5 | 20 | 35 |
| Methylpropandiol | 1,5 | 3,5 | 1,75 | 4 | 3,5 |
| Wasser | ad 100 | ad 100 | ad 100 | ad 100 | ad 100 |

**W/S-Emulsion**

| | 11 | 12 | 13 | 14 | 15 |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Kaliumsorbat | 0,4 | 0,1 | 0,05 | 0,3 | 0,4 |
| Cetyldimethicon | 0,5 | --- | 0,7 | --- | --- |
| Benzylalkohol | --- | --- | 0,05 | --- | 0,1 |
| Pentaerythritol | 0,3 | 6 | 1,25 | 2,0 | 7,5 |
| Icaridin | 3 | 15 | 12,5 | 20 | 7,5 |
| Wasser | ad 100 | ad 100 | ad 100 | ad 100 | ad 100 |

**W/S-Emulsionen**

| | 16 | 17 | 18 | 19 | 20 |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |

(fortgesetzt)

| | 16 | 17 | 18 | 19 | 20 |
|---|---|---|---|---|---|
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s, | q,s, | q,s, | q,s, | q,s, |
| Icaridin | 3,0 | 10 | 12,5 | 15 | 30 |
| Stearyldimethicon | 0,5 | --- | 0,7 | --- | --- |
| Kaliumsorbat | --- | --- | 0,05 | --- | 0,1 |
| Caprylyl Glycol | 0,3 | 2,0 | 12,5 | 1,5 | 3 |
| Wasser | ad 100 | ad 100 | ad 100 | ad 100 | ad 100 |

**W/O-Emulsionen**

| | 21 | 22 | 23 | 24 | 25 |
|---|---|---|---|---|---|
| PEG-22 Dodecyl Glycol Copolymer | 5,0 | 1,5 | 0,25 | --- | 3,0 |
| PEG-45 Dodecyl Glycol Polymer | 1,0 | 1,5 | 1,75 | 3,0 | -- |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Nachtkerzenöl | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Hexandiol | 0,5 | 4 | 1,5 | 2,0 | 10 |
| Icaridin | 5 | 10 | 15 | 20 | 25 |
| Magnesiumsulfat | 0,5 | 0,6 | 0,5 | 0,7 | 1,0 |
| Glycerin | 3,0 | 5,0 | 10,0 | 15,0 | 1,5 |
| Natriumcitrat | 0,2 | 0,1 | --- | --- | --- |
| Zitronensäure | 0,2 | 0,1 | --- | --- | --- |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Benzylalkohol | --- | --- | 0,05 | --- | 0,1 |
| Wasser | ad 100 | ad 100 | ad 100 | ad 100 | ad 100 |

**W/O-Emulsionen**

| | 26 | 27 | 28 | 29 | 30 |
|---|---|---|---|---|---|
| Polyglyceryl-2-dipolyhydroxystearat | 3,0 | --- | 0,25 | --- | 6,0 |
| Polyglyceryl-3 diisostearate | 1,0 | 3,5 | 1,75 | 2,5 | -- |
| PEG-40 sorbitanisostearate | --- | 2,5 | 0,5 | 3,5 | 3,0 |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Ethylhexylglycerin | 1,618 | 1,618 | 0,75 | 1,5 | 4 |
| Icaridin | 1,618 | 16,18 | 7,5 | 15 | 40 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,1 | 0,3 | 1,0 |
| Natriumcitrat | 0,2 | 0,3 | 0,2 | 1,5 | 0.8 |
| Parfum | q,s, | q,s, | q,s, | q,s, | q,s, |
| Capryl-/Caprinsäuretriglycerid | 2,0 | 2,5 | 3,0 | 5,0 | 0,5 |
| Kaliumsorbat | 0,24 | 0,15 | 0,05 | 0,3 | 0,4 |
| Wasser | ad 100 | ad 100 | ad 100 | ad 100 | ad 100 |

**Silikon in Wasser-Emulsion**

| | 31 | 32 | 33 | 34 | 35 |
|---|---|---|---|---|---|
| Dimethiconcopolyol, Caprylic/Caprictriglycerid | 1,0 | 2,0 | 8,0 | 3,0 | 5,0 |
| Cyclomethicon | 12,5 | 15 | 25,0 | 10,0 | 7,5 |
| Dimethicon | 5,0 | 15,0 | 5,0 | 12,0 | 15,0 |
| Mineralöl | 0,5 | 0,75 | 1,0 | 2,0 | 0,25 |
| Phenyltrimethicon | 0,5 | 1,0 | 0,75 | 3,0 | 0,25 |
| Glycerin | 5,0 | 7,5 | 10,0 | 3,0 | 1,0 |
| Decylene Glycol | 1 | 4 | 8 | 4 | 1,5 |
| Icaridin | 10 | 20 | 30 | 40 | 15 |
| Xanthan Gum | --- | 0,1 | -- | 0,25 | 1,0 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Iodopropynylbutylcarbamat | --- | --- | 0,05 | --- | 0,1 |
| Wasser | ad 100 | ad 100 | ad 100 | ad 100 | ad 100 |

**O/W-Emulsion**

| | **36** | **37** | **38** | **39** | **40** |
|---|---|---|---|---|---|
| Glycerylsterat | 1,0 | --- | -- | 0,5 | 0,75 |
| Polyethylenglycol(40)stearat | 10,0 | --- | 5 | -- | -- |
| Triglycerinmethylglucosedistearat | --- | 5,5 | --- | --- | 3,5 |
| Sorbitanstearat | --- | 1,5 | 3 | --- | --- |
| Cyclomethicon | 2,5 | 15 | 8,0 | 5,0 | 7,5 |
| Dimethicon | 5,0 | 3,0 | 5,0 | 2,0 | 5,0 |
| Behenylalkohol | 1 | --- | 2 | 1 | --- |
| Stearylalkohol | --- | 1 | --- | 1 | --- |
| Ginkgo Biloba | 0,1 | 0,5 | 1,0 | 2,5 | 1,0 |
| Icaridin | 7,5 | 15 | 12,5 | 20 | 25 |
| Cetylstearylalkohol | --- | --- | 1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Iodopropynylbutylcarbamat | --- | --- | 0,05 | --- | 0,1 |
| Methylpropandiol | 0,75 | 1,5 | 5 | 2,0 | 10 |
| Wasser | ad 100 | ad 100 | ad 100 | ad 100 | ad 100 |

**O/W-Emulsion**

| | **41** | **42** | **43** | **44** | **45** |
|---|---|---|---|---|---|
| Polyethylenglycol(21)stearylether | 1 | -- | 2,5 | 2 | 1,5 |
| Polyethylenglycol(2)stearylether | 1 | -- | 5,5 | 3 | 7,5 |
| Cetearylglucosid | --- | 8 | --- | --- | --- |
| Behenylalkohol | 3 | 2 | --- | 1 | --- |
| Stearylalkohol | 3 | 2 | --- | 2 | --- |
| Cetylstearylalkohol | 3 | 4 | --- | --- | 2 |
| hydriertes Polyisobuten | 0,5 | 0,75 | 1,0 | 2,0 | 0,25 |
| Icaridin | 10 | 20 | 15 | 7,5 | 5,0 |
| Octyldodecanol | 0,5 | 1,0 | 0,75 | 3,0 | 0,25 |
| Methylpropandiol | 5 | 1,0 | 1,5 | 3 | 7,5 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Iodopropynylbutylcarbamat | --- | --- | 0,05 | --- | 0,1 |

(fortgesetzt)

| | 41 | 42 | 43 | 44 | 45 |
|---|---|---|---|---|---|
| Neopentyl Glycol | 1 | 2 | 1,5 | 3 | 0,5 |
| Wasser | ad 100 | ad 100 | ad 100 | ad 100 | ad 100 |

**O/W-Emulsion**

| | 46 | 47 | 48 | 49 | 50 |
|---|---|---|---|---|---|
| Glycerylsteratcitrat | 1,0 | 0,5 | 1,5 | 9,0 | 0,3 |
| Polyethylenglycol(20)cetearylether | 10,0 | 1,0 | 5 | -- | -- |
| Triglycerinmethylglucosedistearat | --- | --- | 3,5 | --- | 2,5 |
| Trimethylopropane | 0,75 | 1,5 | 2,25 | 12 | 3,75 |
| Icaridin | 7,5 | 15 | 22,5 | 30 | 37,5 |
| Dimethicon | 0,5 | 3,0 | 0,75 | 1,5 | 0,2 |
| Behenylalkohol | 1 | --- | 2 | --- | 0,2 |
| Dicaprylylcarbonat | 3 | 5 | 10 | 5 | 5 |
| Stearylalkohol | --- | --- | --- | 1 | 0,2 |
| Cetylstearylalkohol | --- | --- | 1 | 1 | 0,2 |
| Tocopherol | 0,5 | 0,5 | 0,75 | 0,25 | 0,1 |
| Octyldodecanol | 0,5 | --- | 0,75 | 3,0 | 0,25 |
| Panthenol | 0,5 | --- | 0,75 | 0,25 | 0,1 |
| Carbomer | 0,05 | 0,35 | 0,15 | 0,1 | --- |
| Parfum | q,s, | q,s, | q,s, | q,s, | q,s, |
| Caprylic/Capric Triglycerid | 1 | 5 | 3 | 5 | 10 |
| Methylparaben | 0,4 | 0,3 | 0,05 | 0,3 | 0,4 |
| Propylparaben | 0,3 | --- | 0,25 | 0,15 | --- |
| Iodopropynylbutylcarbamat | --- | --- | 0,05 | --- | 0,1 |
| Phenoxyethanol | --- | 0,5 | --- | 0,15 | --- |
| Sorbitol | 10 | --- | -- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

**O/W-Emulsion**

| | 51 | 52 | 53 | 54 | 55 |
|---|---|---|---|---|---|
| Glycerylsterat | 2,6 | --- | --- | 2,7 | 2,6 |
| Caprylic/Capric Triglycerid | 3,5 | 4,0 | 3,75 | 3,1 | 5,5 |
| Dicaprylylcarbonat | 3,5 | 2,5 | 3,75 | --- | 3,0 |
| Triglycerinmethylglucosedistearat | --- | 3,0 | 3,0 | --- | --- |

(fortgesetzt)

| | 51 | 52 | 53 | 54 | 55 |
|---|---|---|---|---|---|
| Cetystearylalkohol | 3,0 | --- | --- | 4,0 | 3,5 |
| Dimethicon | --- | 3,0 | 5,0 | 3,0 | 2,75 |
| Cetylalkohol | --- | 3,0 | 1,5 | 1,25 | --- |
| Polyethylenglycol(40)stearat | 0,8 | --- | --- | 1,3 | 1,3 |
| C12-15 Alkylbenzoat | --- | 2,5 | 3,75 | 3,0 | 4,5 |
| Buthylethylpropandiol | 0,5 | 1,0 | 1,5 | 4 | 2,5 |
| Icaridin | 5 | 10 | 15 | 20 | 25 |
| Natriumoleanolat | 0,1 | 0,1 | 0,1 | 0,075 | 0,1 |
| Cyclomethicon | 3,5 | --- | --- | --- | 0,25 |
| Propylenglykol | 0,5 | 0,5 | 0,5 | 0,35 | 0,2 |
| Mineralöl | --- | --- | --- | 6,0 | --- |
| Phenoxyethanol | 0,4 | 0,4 | 0,4 | 0,75 | 1,25 |
| Zitronensäure | --- | --- | 0,05 | --- | --- |
| Ethanol | --- | --- | --- | 3,0 | --- |
| Ethylparaben | --- | --- | --- | 0,3 | --- |
| Methylparaben | 0,2 | 0,2 | 0,2 | 0,2 | 0,2 |
| Glycerin | 10,0 | 10,0 | 10,0 | 10,0 | 12,5 |
| Propylparaben | 0,1 | 0,1 | 0,1 | 0,1 | 0,1 |
| Carbomer, Natrium-Salz | 0,1 | 0,1 | 0,1 | 0,2 | 0,1 |
| Parfum | q,s, | q,s, | q,s, | q,s, | q,s, |
| Iodopropynylbutylcarbamat | 0,018 | 0,018 | 0,018 | --- | 0,018 |
| Wasser | ad 100 | ad 100 | ad 100 | ad 100 | ad 100 |

**Pumpspray**

| | 56 | 57 | 58 | 59 | 60 |
|---|---|---|---|---|---|
| Polyethylenglycol(40)stearat | 1,0 | --- | 0,5 | -- | -- |
| Cyclomethicon | 0,5 | --- | --- | --- | --- |
| Icaridin | 2 | 10 | 15 | 20 | 40 |
| Parfum | q,s, | q,s, | q,s, | q,s, | q,s, |
| Ethanol | 15 | 20 | 30 | 40 | 20 |
| Methylparaben | 0,4 | 0,1 | 0,05 | 0,3 | 0,4 |
| Meeresextrakt | --- | --- | 0,1 | --- | --- |
| Aloe Vera Extrakt | 0,1 | --- | 0,1 | --- | --- |
| Hammamelis-Extrakt | 0,1 | --- | 0,1 | 5 | 0,5 |
| Glycerin | 5 | 10 | 3 | 15 | 7,5 |
| Di-Trimethylolpropane | 1,0 | 2,5 | 3,5 | 0,15 | 7,5 |
| Wasser | ad 100 | ad 100 | ad 100 | ad 100 | ad 100 |

**Spray mit Repellent**

| | 61 | 62 | 63 | 64 | 65 |
|---|---|---|---|---|---|
| Ethylbutylacetylaminopropionat | 5 | 35 | 15 | 20 | 7,5 |
| N, N-Diethyltoluolamid | 5 | --- | -- | 1 | -- |
| Icaridin | 5 | 3,0 | 2,0 | 1,5 | 12,5 |
| 1-(3-Cyclohexen-1-yl-carbonyl)-2-methylpiperidin | 5 | --- | 2 | 1 | --- |
| Hammamelis-Extrakt | 0,2 | --- | --- | 1 | 0,2 |
| Aloe Vera Extrakt | --- | --- | 1 | 1 | 5,0 |
| Tocopherol | 0,5 | 0,5 | 0,75 | 0,25 | 0,1 |
| Meeresextrakt | 0,5 | --- | 0,75 | 3,0 | 0,25 |
| Panthenol | 0,5 | --- | 0,75 | 0,25 | 0,1 |
| Ethylhexylglycerin | 1 | 1,5 | 0,5 | 0,75 | 10 |
| Parfum | q,s, | q,s, | q,s, | q,s, | q,s, |
| Sorbitol | 10 | --- | -- | 5 | --- |
| Butylenglykol | --- | --- | --- | 5 | 10 |
| Propylenglykol | --- | --- | 10 | 5 | --- |
| Glycerin | --- | 7,5 | --- | --- | --- |
| Wasser | ad 100 | ad 100 | ad 100 | ad 100 | ad 100 |

## Patentansprüche

1. Packmittel aus Polyethylen hoher Dichte (HOPE), enthaltend eine kosmetische Zubereitung enthaltend

   a) 1-Piperidincarboxylsäure 2-(2-hydroxyethyl)-1-methylpropylester (INN: Icaridin) und
   b) eine oder mehrere Substanzen mit einem log P-Wert von -2,5 bis +2,5, gewählt aus der Gruppe mehrwertige Alkohole, lineare Alkohole, verzweigte Alkohole, C5-C10 Alkylalkohole, Polyglycerine.

2. Packmittel nach Anspruch 1, **dadurch gekennzeichnet, dass** die kosmetische Zubereitung 1-Piperidincarboxylsäure 2-(2-hydroxyethyl)-1-methylpropylester (INN: Icaridin) und ggf. weitere Repellent-Wirkstoffe mit einem Dampfdruck von mindestens 3,2 $10^{-4}$ hPa bei 20 °C in einer Gesamtkonzentration von 1 bis 40 Gewichts-%, bezogen auf das Gesamtgewicht der Zubereitung, enthält.

3. Packmittel nach einem der vorhergehenden Ansprüche, **dadurch gekennzeichnet, dass** die kosmetische Zubereitung Substanzen mit einem log P-Wert von -2,5 bis +2,5 in einer Gesamtkonzentration von 0,1 bis 20 Gewichts-%, bezogen auf das Gesamtgewicht der Zubereitung, enthält.

4. Packmittel nach einem der vorhergehenden Ansprüche, **dadurch gekennzeichnet, dass** das Gewichtsverhältnis der Gesamtmenge an Repellent-Wirkstoffen mit einem Dampfdruck von mindestens 3,2 $10^{-4}$ hPa bei 20 °C zur Gesamtmenge an Substanzen mit einem log P-Wert von -2,5 bis +2,5 von 20 : 1 bis 1 : 1 beträgt.

5. Packmittel nach einem der vorhergehenden Ansprüche, **dadurch gekennzeichnet, dass** die kosmetische Zubereitung frei ist von Parabenen.

## Claims

1. Packaging means made of high-density polyethylene (HDPE), comprising a cosmetic preparation comprising

a) 2-(2-hydroxyethyl)-1-methylpropyl 1-piperidine carboxylate (INN: Icaridin) and
b) one or more substances with a log P value of from -2.5 to +2.5, selected from the group of polyhydric alcohols, linear alcohols, branched alcohols, C5-C10 alkyl alcohols, polyglycerols.

**2.** Packaging means according to Claim 1, **characterized in that** the cosmetic preparation comprises 2-(2-hydroxyethyl)-1-methylpropyl 1-piperidine carboxylate (INN: Icaridin) and optionally further repellent active ingredients with a vapour pressure of at least $3.2 \cdot 10^{-4}$ hPa at 20°C in a total concentration of 1 to 40% by weight, based on the total weight of the preparation.

**3.** Packaging means according to one of the preceding claims, **characterized in that** the cosmetic preparation comprises substances with a log P value of from -2.5 to +2.5 in a total concentration of from 0.1 to 20% by weight, based on the total weight of the reparation.

**4.** Packaging means according to one of the preceding claims, **characterized in that** the weight ratio of the total amount of repellent active ingredients with a vapour pressure of at least $3.2 \cdot 10^{-4}$ hPa at 20°C to the total amount of substances with a log P value of from -2.5 to +2.5 is from 20:1 to 1:1.

**5.** Packaging means according to one of the preceding claims, **characterized in that** the cosmetic preparation is free from parabens.

**Revendications**

**1.** Emballage en polyéthylène haute densité (HDPE), contenant une préparation cosmétique contenant

a) du 1-pipéridinecarboxylate de 2-(2-hydxoxyéthyl)-1-méthylpropyle (INN : icaridine) et
b) une ou plusieurs substances ayant une valeur log P de -2,5 à +2,5, choisies dans le groupe constitué par des alcools polyhydriques, des alcools linéaires, des alcools ramifiés, des alcools alkyliques en $C_5$-$C_{10}$, des polyglycérols.

**2.** Emballage selon la revendication 1, **caractérisé en ce que** la préparation cosmétique contient du 1-pipéridinecarboxylate de 2-(2-hydroxyéthyl)-1-méthyl-propyle (INN : icaridine) et éventuellement d'autres substances actives répulsives ayant une tension de vapeur d'au moins $3{,}2.\ 10^{-4}$ hPa à 20 °C, à une concentration totale de 1 à 40 % en poids, par rapport au poids total de la préparation.

**3.** Emballage selon l'une quelconque des revendications précédentes, **caractérisé en ce que** la préparation cosmétique contient des substances ayant une valeur log P de -2,5 à +2,5, à une concentration totale de 0,1 à 20 % en poids, par rapport au poids total de la préparation.

**4.** Emballage selon l'une quelconque des revendications précédentes, **caractérisé en ce que** le rapport pondéral de la quantité totale de substances actives répulsives ayant une tension de vapeur d'au moins $3{,}2.\ 10^{-4}$ hPa à 20 °C à la quantité totale de substances ayant une valeur log P de -2,5 à +2,5 vaut de 20:1 à 1:1.

**5.** Emballage selon l'une quelconque des revendications précédentes, **caractérisé en ce que** la préparation cosmétique est exempte de parabens.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10349666 A1
- WO 03020232 A
- WO 0243490 A
- WO 0243656 A


### In der Beschreibung aufgeführte Nicht-Patentliteratur


- **Rekker ; Mannhold.** Calculation of Drug Lipophilicity. Verlag Chemie Weinheim, 1992
- **Kubiny, H.** *QSAR: Hansch Analyses and Related Approaches,* vol. 1
- **Brehm, O. ; Ohlmeyer, I ; Fels, G.** *Automatisierte pKa- und logP-Bestimmung. GIT Labor-Fachzeitschrift,* 1997, vol. 41, 368-374